Europäisches Patentamt

European Patent Office

Office européen des brevets

(11) Publication number: **0 476 730 A1**

## EUROPEAN PATENT APPLICATION

(21) Application number: **91202094.8**

(22) Date of filing: **16.08.91**

(51) Int. Cl.5: **A61B 10/00, A61B 5/00**

(30) Priority: **27.08.90 NL 9001877**

(43) Date of publication of application:
**25.03.92 Bulletin 92/13**

(84) Designated Contracting States:
**DE ES FR GB IT NL**

(71) Applicant: **HOLLANDSE SIGNAALAPPARATEN B.V.**
**Zuidelijke Havenweg 40 P.O. Box 42**
**NL-7550 GD Hengelo(NL)**

(72) Inventor: **Franke, Gerhardus Bonifatius**
**Gerst 6**
**NL-7623 HJ Borne(NL)**

(54) **Temperature measuring system.**

(57) The system for ovulation detection and prediction comprises an implanted or embedded temperature sensor (1) and a processing unit (2) provided with a transmitter unit (3), a transmitting antenna (4), a receiving antenna (5), a receiver unit (6), a processor (7) and a clock unit (8).

A harmonics generator is used as the temperature sensor, enabling the transmitter unit (3) to operate on a very low power level.

Fig. 1

The invention relates to a temperature measuring system to determine the moment when in a woman an ovulation occurs, employing a temperature sensor introduced into the woman's body, and a processor that processes temperature parameters obtained through the temperature sensor so as to determine the moment described above.

The operation is based on the fact that the body temperature of a woman rises at the time of the ovulation. This rise in temperature is merely several tenths of a degree celsius and is easily masked by other factors. Therefore it is common practice to refer to the basal temperature which is the body temperature measured before getting up, after at least six hours of sleep, at least ten hours after a meal. By measuring this basal temperature daily, the moment of the ovulation can be determined accurately. When these daily measurements are continued for several months, the ovulation can also be predicted.

Knowing the moment of the ovulation is of great importance. It helps couples who are childless against their will to determine the optimum moment for conception. Avoiding pregnancy is possible by renouncing sexual intercourse just before or during the ovulation. Furthermore, it is assumed, on the basis of statistical analyis, that the actual time of having sexual intercourse also determines the sex of a child. Sexual intercourse two days before the ovulation results statistically in the birth of more girls, while sooner before the ovulation it results in more boys. Also on the basis of statistical analysis, sexual intercourse is advised against soon after the ovulation as this increases the likelihood of Down's syndrome in the child.

A temperature measuring system to determine the basal temperature of a woman is known from the NL-A 80.06012.

The temperature measuring system and combined registration unit described in the application can be placed on the cervix of the woman by a gynaecologist and can be removed after, for instance, a few months, after which the registration unit can be read. In this way it is possible to determine at which moments in that length of time an ovulation took place in the woman. This information can be of use to cases of undesired childlessness.

For a wider application of the temperature measuring system, for instance to prevent unwanted pregnancy, the repeated inserting, removing and reading of that temperature measuring system is inconvenient. The present temperature system according to the invention removes this drawback.

It is characterised in that the processor is placed outside the body of the woman.

An additional advantage of the temperature measuring system according to the invention is that the ovulation can be predicted on the basis of very recent measurements. This is very important, because a higher body temperature, for instance as a result of an illness, can cause the ovulation to occur earlier. This aspect can be taken into account in the model employed by the processor. Likewise a delayed ovulation, which is a known phenomenon, can be detected on the basis of recent measurements, such that the risk of an unwanted pregnancy is very small.

Finally, using the temperature measuring system according to the invention has the advantage that a pregnancy can be ascertained with near absolute certainty sixteen days after the ovulation. If there is no pregnancy, the temperature will drop during that time to the value it had before the ovulation. In the event of pregnancy the temperature remains high.

The invention viii now be described in further detail with reference to the accompanying figures, of which:

Fig. 1 presents a block diagram of a possible embodiment of a temperature measuring system according to the invention;

Fig. 2 presents a first embodiment of a temperature sensor;

Fig. 3 presents a second embodiment of a temperature sensor;

Fig. 4 presents a diagram of the strength of the radiation field E emitted by a harmonic generator as a function of the frequency F of the primary radiation field.

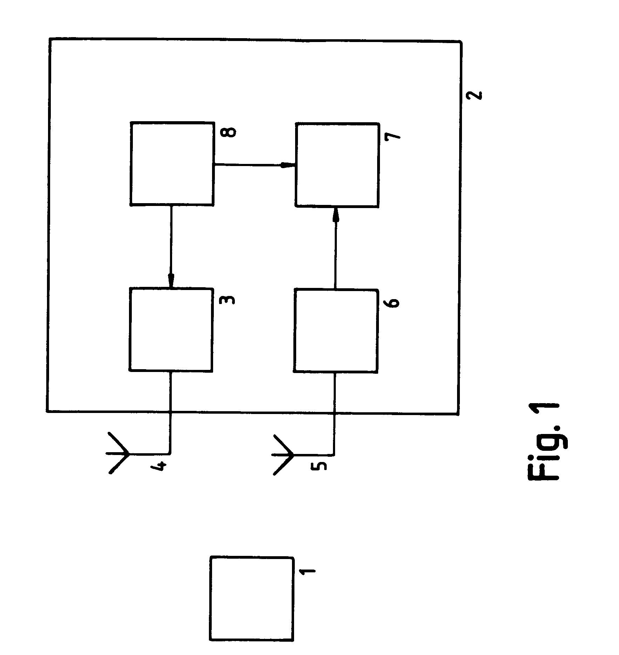

In fig. 1 a block diagram is presented of a possible embodiment of the invention. A temperature sensor 1 is accommodated in the body of a woman. A processing unit 2 contains a transmitter unit 3, a transmitting antenna 4, a receiving antenna 5, a receiver unit 6, a processor 7 and a clock unit 8.

Periodically, for instance at six o'clock a.m., the clock unit 8 activates transmitter unit 3, which through transmitting antenna 4 briefly emits electromagnetic energy. Part of the emitted energy impinges on the temperature sensor 1, and is partly re-emitted by it. The re-emitted energy is provided with a parameter which corresponds with the temperature of temperature sensor 1. Part of the re-emitted energy impinges on receiving antenna 5 and is processed by receiver unit 6 to form an input signal for processor 7. Processor 7, which usually is an A/D converter followed by a microcomputer, can determine and predict, using a known method, the moment of the ovulation on the basis of the periodical measurements and of programmed foreknowledge. Processor 7 will normally be provided with indication means, which indicate if an ovulation is about to occur, in other words if the woman is fertile at the moment. To prevent processor 7 from responding to interference signals, it will only accept an input signal when transmitter unit 3 is activated.

The processing unit can be placed in the bedroom near the bed, the range of the temperature measuring system then needing to be only a few metres. The advantage is that very little electromagnetic energy has to be emitted.

It is usual for the temperature to be measured after the woman has slept for at least six hours. The embodiment described makes it possible to determine when the woman gets into bed. To this end the clock unit 8 can activate transmitter unit 3 at one hour intervals starting from, for instance, ten o'clock p.m. As soon as transmitter unit 6 detects energy re-emitted by temperature sensor 1, the conclusion is drawn that the woman has gone to bed. On this basis the optimum time for the actual temperature measurement can be determined.

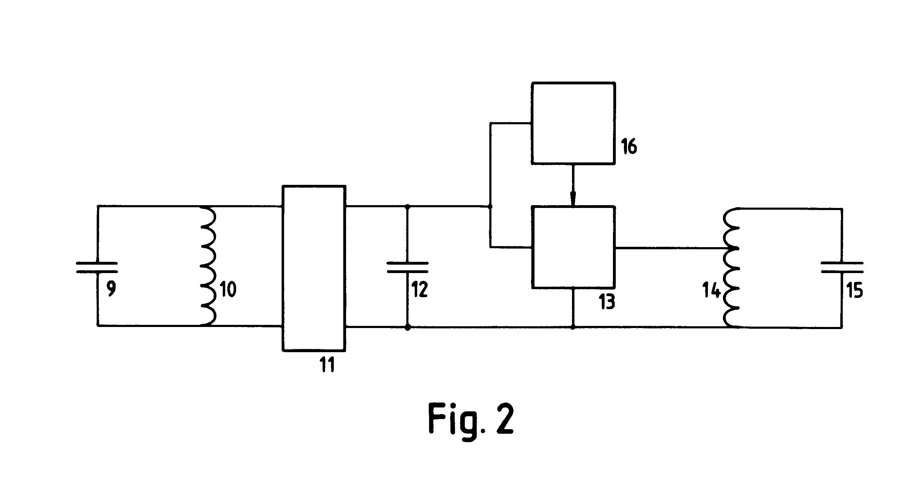

In a first embodiment of the invention the temperature sensor 1 is implemented as presented in fig. 2. Capacitor 9 and coil 10 form a first circuit tuned to a frequency $F_o$. When temperature sensor 1 is brought within a radiation field of frequency $F_o$ generated by a transmitter unit 3, a rectifier circuit 11 will charge a buffer capacitor 12. Temperature sensor 1 contains transmitting means, formed by an oscillator 13 and a second circuit formed by coil 14 and capacitor 15. The transmitting means obtain their supply voltage from buffer capacitor 12. The frequency $F_r$ of the signal re-emitted by the transmitting means is determined practically exclusively by the second circuit, always observing that $F_r \neq F_o$.

In a first special embodiment, the second circuit is dimensioned such that $F_r$ is dependent on the temperature of the temperature sensor 1, for instance through providing capacitor 15 with a dielectric possessing a strongly temperature-dependent dielectric constant. The temperature-dependent parameter then is $F_r$, the frequency of the signal re-emitted by the transmitting means. Receiver unit 6 shall be equipped with means to translate the frequency of the received signal into a voltage which is proportional to it. An FM demodulation circuit well-known in the field can be employed here.

In a second special embodiment, a modulator 16 is added with which the signal re-emitted by the transmitting means can be modulated. Modulator 16, too, obtains its supply voltage from buffer capacitor 12. Modulator 16 can, for instance, effect a pulse-shaped modulation, whereby the length of the pulse depends on the temperature. For this purpose, modulator 16 can comprise a monostable multivibrator, whose time-determining resistance is strongly temperature dependent. Also, a series of pulses can be emitted, whereby the interpulse interval varies with the temperature. The pulses can be realised through amplitude modulation, but also through frequency modulation by connecting or not connecting a capacitor in parallel to capacitor 15.

It is also possible for modulator 16 to be provided with a circuit which determines the temperature and outputs it as a digital value in the manner known from digital clinical thermometers. Modulator 16 modulates the re-emitted signal such that the digital value is re-emitted as a series of data bits. To the series of data bits can then be added a few bits as an identification code. This further reduces the risk, which is small in the first place, of processor 7 processing a temperature measurement originating from another temperature sensor which happened to be within the radiation field of transmitting antenna 4.

Receiver unit 6 needs to be equipped with a demodulator circuit, suited to the type of modulation used. Processor 7 can convert the series of data bits to the original value and is also able to check the identification code.

Finally, it can be noted that in the embodiment described with reference to fig. 2 the second tuned circuit can be replaced by an ultrasonic transducer which, in one of the previously described ways, can emit pulses in which the temperature of the temperature sensor 1 is present in coded form. Receiver antenna 5 then is replaced by a microphone suitable for the reception of ultrasonic oscillations.

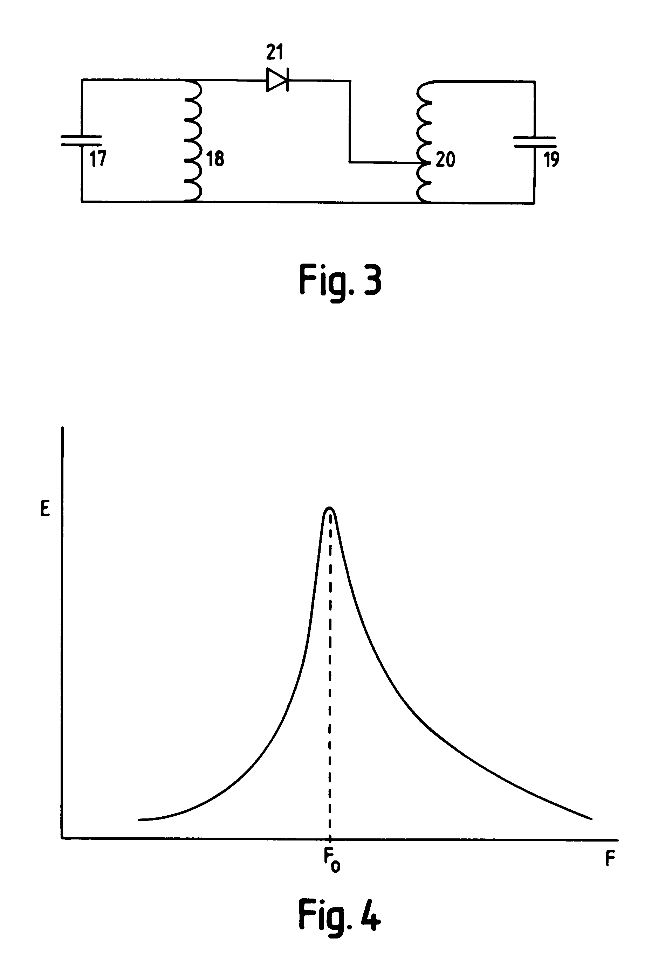

In an alternative embodiment of the invention, allowing the transmitter unit 3 to operate on a very low power level, the temperature sensor 1 is implemented as a harmonic generator, as shown in fig. 3. Capacitor 17 and coil 18 form a first circuit, tuned to a frequency $F_o$. Capacitor 9 and coil 20 form a second circuit, tuned to $nF_o$ (n = 2, 3, ....). Diode 21 forms a non-linear element which, when the first circuit oscillates at a frequency F, causes the second circuit to oscillate at a frequency nF. When a harmonic generator of this type is brought into a primary radiation field of frequency F, the second circuit will generate a radiation field of frequency nF. If the frequency of the primary radiation field differs from the frequency $F_o$, the second circuit will generate practically no radation field. This is graphically represented in fig. 4, the strength of the radiation field E generated by the second circuit being plotted as a function of the frequency F of the primary radiation field into which the harmonic generator has been brought.

Without special provisions, a harmonic generator is practically unsusceptible to temperature. Provisions have therefore to be made to achieve susceptibility to temperature. This can be done, for instance, through tuning the second circuit to a relatively narrow band and, moreover, providing capacitor 19 with a dielectric possessing a strongly temperature-dependent dielectric constant. A suitable dielectric, for instance, is ceramics with which a temperature dependence of 1% per °C can be achieved for the dielectric constant. Alternatively, coil 20 can be provided with a ferromagnetic core, which is selected such that the Curie point, at which the ferromagnetic proporties are strongly temperature dependent, is approximately 37°C.

If a relatively narrow band and temperature-dependent second circuit is chosen, the first circuit needs to be tuned to a relatively wide band, such that a resonance peak as shown in fig. 4 is substantially exclusively dependent on the second circuit.

It is, however, also possible for both circuits to be implemented as relatively narrow-band ones, the first circuit as well as the second being made temperature dependent. Then, an added condition is that for each temperature in the required temperature range of, usually, 36°C to 38°C, the resonant frequencies of both circuits match if the factor n is taken into consideration.

With the temperature sensor 1 implemented as a harmonic generator, the transmitter unit 3 needs to be equipped with a generator which, under the control of the processor 7, can emit a number of frequencies $f_i$ around $F_o$. Simultaneously the receiver unit 6 needs to be matched, under the control of the processor 7, to the associated frequencies $nF_i$. In this way the resonance curve according to fig. 4 of the temperature-dependent circuit can be measured, resulting in the position of the resonance peak and hence the temperature of the temperature sensor being determined. For the generator, a digital crystal-controlled synthesizer is particularly suitable, on the one hand because it is directly controllable by a microcomputer, on the other because it possesses the required stability for an accurate measurement.

When a harmonic generator is employed, transmitter unit 3 needs to be provided with a first filter which passes on, to the transmitting antenna 4, only signals of a frequency close to $F_o$. Without this filter, harmonics of a frequency close to $F_o$ generated in, for instance, the output stage of the transmitter unit 3, could directly penetrate into the receiver unit 4, influencing the measurement. Likewise, receiver unit 4 needs to be provided with a second filter which passes on only signals of a frequency close to $nF_o$ from the receiving antenna. Without this second filter, strong signals of a frequency close to $F_o$ could penetrate into the receiver unit 4 and there generate harmonics, which would influence the measurement.

The first filter and the second filter can be combined into a diplexer well-known in the field, permitting transmitting antenna 4 and receiving antenna 5 to be combined to a single antenna.

In this embodiment, the processing unit 2 is particularly suitable for incorporation into a clock radio, whereby the antenna, the synthesizer, the clock unit and the processor could function as part of the clock radio and simultaneously as part of the temperature measuring system.

Of course, processing unit 2 can also be combined with other obvious objects. Also, processing unit 2 can be implemented as an insert card or as an interface for a computer, providing additional recording and display possibilities.

**Claims**

1. Temperature measuring system to determine the moment when in a woman an ovulation occurs, comprising a transmitter unit for the generation of a first radio frequency electromagnetic field, in which the woman can be placed, the woman being provided with an implanted or embedded temperature sensor comprising antenna means, receiver means for receiving the first radio frequency electromagnetic field and transmitter means coupled to the receiver means; a receiver unit for receiving a second radio frequency electromagnetic field generated by the transmitter means and a processor connected to the receiver unit for the processing of received signals, characterised in that the temperature sensor comprises a resonant harmonics generator for converting at least part of the energy of the first radio frequency electromagnetic field in the second radio frequency electromagnetic field, and that the resonant frequency of the harmonics generator is temperature dependent.

2. Temperature measuring system according to claim 1, characterised in that the resonant harmonics generator is provided with two resonant circuits, coupled with a non-linear element.

3. Temperature measuring system according to claim 2, characterised in that at least one resonant circuit comprises a temperature dependent capacitor.

4. Temperature measuring system according to claim 2, characterised in that the non-linear element is a diode.

5. Temperature measuring system according to one of the above claims, characterised in that the transmitter unit is provided with a digital, crystal-controlled synthesizer for the generation of a set of transmitter frequencies, situated around the resonant frequency of the harmonics generator.

6. Temperature measuring system according to one of the above claims, characterised in that the transmitter unit is provided with a first filter, placed at the output of the transmitter unit, which does not pass - at least substantially - harmonics of the transmit frequency.

7. Temperature measuring system according to claim 6, characterised in that the receiver unit is provided with a second filter, placed at the input of the receiver unit, which does not pass - at least substantially - the transmit signal of the transmitter unit.

8. Temperature measuring system according to claim 7, characterised in that the first filter and the second filter are combined as a diplexer, provided with a single antenna connection.

EP 0 476 730 A1

**Fig. 1**

Fig. 2

Fig. 3

Fig. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE-A-3 342 251   (WEILAND)<br>* page 7, line 27 - page 8, line 5 * * <br>– – – | 1 | A 61 B 10/00<br>A 61 B 5/00 |
| A | US-A-3 656 132   (BRUMBELOW)<br>* abstract * * <br>– – – | 1 | |
| A | US-A-4 075 632   (BALDWIN)<br>* column 6, line 31 - line 39; figure 3 * * * column 6, line 60 - column 7, line 4 * * <br>– – – | 1 | |
| A | GB-A-2 016 146   (SVEJSECENTRALEN)<br>* page 1, line 76 - line 79; figure 2 * * <br>– – – | 1 | |
| A | EP-A-0 195 207   (FROHN)<br>* claim 1 * * <br>– – – | 1 | |
| A | US-A-3 893 111   (COTTER)<br>* abstract * * <br>– – – | 1 | |
| A | DE-A-3 219 558   (KOSTER)<br>– – – – – | | |

**TECHNICAL FIELDS SEARCHED (Int. Cl.5)**

A 61 B

The present search report has been drawn up for all claims

| Place of search | Date of completion of search | Examiner |
|---|---|---|
| The Hague | 25 November 91 | BARTON S.A. |